# EUROPEAN PATENT APPLICATION

(11) **EP 4 687 151 A1**
(43) Date of publication of application: **04.02.2026**
(21) Application number: 25193586.2
(22) Date of filing: 01.08.2025
(51) Int. Cl.: G16H 20/40, A61C 13/00

(54) **SYSTEM, APPARATUES, AND METHODS FOR CREATING DIGITAL DENTAL PROSTHESIS MODELS**

(30) Priority: 01.08.2024 US 202418791676
(71) Applicant: DENTSPLY SIRONA Inc., York, PA 17401 (US)
(72) Inventor: DERZAPF, Evgenij, 64653 Lorsch (DE); KOZA, André, 68163 Mannheim (DE); SCHNEIDER, Sascha, 64367 Mühltal (DE); BIELSER, Daniel, 3612 Steffisburg (CH); WEY, Peter, 5436 Würenlos (CH); LÖSCHHORN, Sandro, 8172 Niederglatt (CH); MUFF, Samuel, 8713 Uerikon (CH); TANAKA, Simon, 1010 Lausanne (CH)
(74) Representative: Aldridge, Henry Alexander

(57) **Abstract**

Apparatuses, systems, and methods are provided for creating a digital model of a dental prosthesis. Information about the dental prosthesis to be modeled is received, and an initial model of the dental prosthesis is generated based on the received information. Information for modifying the initial model of the dental prosthesis is received. A modified model of the dental prosthesis is generated based on the information for modifying the initial model of the dental prosthesis. At least one of the information about the dental prosthesis and the information for modifying the initial model of the dental prosthesis is received by the computer system in the form of voice or text. Artificial intelligence can be used in at least one of the generation of the initial model of the dental prosthesis and the generation of the modified model of the dental prosthesis.

## Description

### BACKGROUND

### Field of the Invention

Embodiments of the invention relate to systems, apparatuses, and methods for creating a digital model of a dental prosthesis. Embodiments of the invention also relate to systems, apparatuses, and methods for creating a digital model of a dental prosthesis with the assistance of artificial intelligence.

### Related Art

Dental restoration procedures, such as the placement of crowns, are commonplace in dental care due to their pivotal role in preserving and enhancing oral health. Dental restoration processes involve careful assessment, preparation, and customization of a dental prosthesis to achieve a precise fit and natural appearance. Given their versatility and effectiveness, dental professionals frequently recommend and perform prosthesis placements to address a range of dental issues, promoting both the longevity of the tooth and the overall well-being of the patient.

Creating a design for a dental prosthesis such as a crown involves a systematic and precise process. After a thorough examination of the patient's oral condition, the dentist typically takes impressions or digital scans of the affected tooth and surrounding structures. Using the impression model, the dental professional plans the shape, size, and contours of the dental prosthesis. Such a plan must carefully consider factors such as the patient's bite, alignment, and aesthetic preferences. In some cases, to create a model from which the dental prosthesis will be fabricated digital technologies like CAD/CAM (computer-aided design/computer-aided manufacturing) are employed. But such technologies are not easy for the dental professional to use-many dental professionals find using such technologies difficult and time-consuming.

Once a dental prosthesis model is finalized, it guides the fabrication of the dental prosthesis, whether through traditional methods involving a dental lab or advanced in-office milling machines. The goal is to create a custom prosthesis that not only restores the tooth's functionality and strength but also seamlessly blends with the natural dentition for a cosmetically pleasing result.

### SUMMARY OF THE INVENTION

According to one embodiment, a system is provided for generating a digital model of a dental prosthesis. The system comprises at least one processor configured to read out and execute instructions stored in at least one memory to thereby cause the system to function as a first receiving unit configured to receive information about the dental prosthesis to be modeled; an initial dental prosthesis model generation unit configured to generate an initial model of the dental prosthesis using the received information about the dental prosthesis to be modeled; a second receiving unit configured to receive information for modifying the initial model of the dental prosthesis; and a modified dental prosthesis model generation unit configured to generate a modified model of the dental prosthesis using the information for modifying the initial model of the dental prosthesis. The system is configured to receive at least one of the information about the dental prosthesis to be modeled and the information for modifying the initial model of the dental prosthesis in the form of voice or text. At least one of the first receiving unit, initial dental prosthesis model generation unit, second receiving unit, and modified dental prosthesis model generation unit is configured to use artificial intelligence when performing functions.

According to another embodiment, a system is provided for generating a digital model of a dental prosthesis. The system comprises at least one processor configured to read out and execute instructions stored in at least one memory to thereby cause the system to function as a receiving unit configured to receive information in the form of voice or text about the dental prosthesis to be modeled, and a dental prosthesis model generation unit configured to generate a model of the dental prosthesis based on the information received by the receiving unit, with the model generation unit using artificial intelligence.

According to another embodiment, a method performed by a computer system is provided for generating a digital model of a dental prosthesis. The method comprises receiving information about the dental prosthesis to be modeled; generating an initial model of the dental prosthesis based on the received information about the dental prosthesis to be modeled; providing a display of the initial model of the dental prosthesis on a display device; receiving information for modifying the initial model of the dental prosthesis; generating a modified model of the dental prosthesis based on the information for modifying the initial model of the dental prosthesis; and providing a display of a modified model of the dental prosthesis on the display device. At least one of the information about the dental prosthesis and the information for modifying the initial model of the dental prosthesis is received by the computer system in the form of voice or text. Artificial intelligence is used in at least one of the generation of the initial model of the dental prosthesis and the generation of the modified model of the dental prosthesis.

According to another embodiment, provided is a non-transitory computer readable storage medium storing computer-readable instruction that cause a processor of a computer system to receive information about the dental prosthesis to be modeled; generate an initial model of the dental prosthesis based on the received information; provide a display of the initial model of the dental prosthesis on a display device; receive information for modifying the initial model of the dental prosthesis; generate a modified model of the dental prosthesis based on the information for modifying the initial model of the dental prosthesis; and provide a display of a modified model of the dental prosthesis on the display device. At least one of the information about the dental prosthesis and the information for modifying the initial model of the dental prosthesis is received by the computer system in the form of voice or text. Artificial intelligence is used in at least one of the generation of the initial model of the dental prosthesis and the generation of the modified model of the dental prosthesis.

### BRIEF DESCRIPTION OF THE DRAWINGS

FIG. 1 is conceptual diagram of a system according to embodiments of the invention.
FIG. 2 is a flow chart showing steps of methods according to embodiments of the invention.
FIGS. 3A-3G show examples of modifications to a dental prosthesis model according to embodiments of the invention.
FIGS. 4A-4E show examples of modifications to another dental prosthesis model according to embodiments of the invention.

### DETAILED DESCRIPTION OF THE INVENTION

Embodiments of the invention will now be described. The embodiments include systems, apparatuses, and methods that for creating a digital model of a dental prosthesis. Particular embodiments of invention include the use of artificial intelligence (AI) in generating digital model of the dental prosthesis based received speech or text descripting the dental prosthesis to be created.

As used herein, a "dental prosthesis" refers to an artificial structure designed to replace missing teeth to restore oral function, aesthetics, and/or facial appearance. Examples of dental prostheses used in dental restorations include crowns, bridges, dentures, dental implants, and veneers. For convenience, many of the descriptions below will be in the specific context of the creation of a crown. But, as evident from the disclosure, embodiments of the invention are not limited to the design of crowns.

As will be discussed more fully below, embodiments of the invention include the use of AI in the generation of digital models of dental prostheses. As will be appreciated by those skilled in the art, such AI encompasses a diverse array of technologies and methodologies. For example, AI systems may use large language models to comprehend and interpret textual or vocal data, extracting meaningful insights and context. These models are created using large amounts of training data. The AI may also use advanced algorithms to convert textual or vocal information into actionable formats, enabling seamless integration with digital workflows. With respect to the three-dimensional modeling of dental prostheses, the AI may employ specialized algorithms to interpret and convert vocal or textual inputs into intricate digital representations. By employing dedicated models tailored for representing dental prostheses, the AI can generate detailed and realistic digital models of the dental prostheses. Further details of the AI, and how the AI may be trained, will be provided below.

In particular embodiments of the invention, the AI system receives text or speech as input and generates or modifies models of dental restorations as output. The AI system may include a large language model, which interprets the meaning of the users' specified requirements and composes a solution directly in a three-dimensional model. For this purpose, the AI system may include a large language model and a second model that is designed for producing three-dimensional models of dental prosthesis as an output. As such, the AI system is able to recognize key words and assemble matching shapes together, and the AI system can also develop a complex understanding of a prosthesis shapes and of how the shaped interacts with the surrounding neighboring and antagonistic teeth.

In embodiments of the invention, the AI system may be trained with prosthesis proposals, wherein the AI system is given tuples of speech and/or text and the geometry of prosthesis that are a good representation of the speech and/or text. In embodiments of the invention, the AI system may also be trained based on modifications to existing models of prosthesis, wherein the AI system is the existing model as input and is given the geometry best representing the requested modification. For both training with prosthesis proposals and prosthesis modifications, the initial training sets may be generated artificially. Then, once a starting AI system is developed, a large number of training sets can be input to the AI system through daily work of some dental professionals by storing for their speech and/or text input with the AI geometry of the prosthesis model and also the final prosthesis model that has manually adapted geometry.

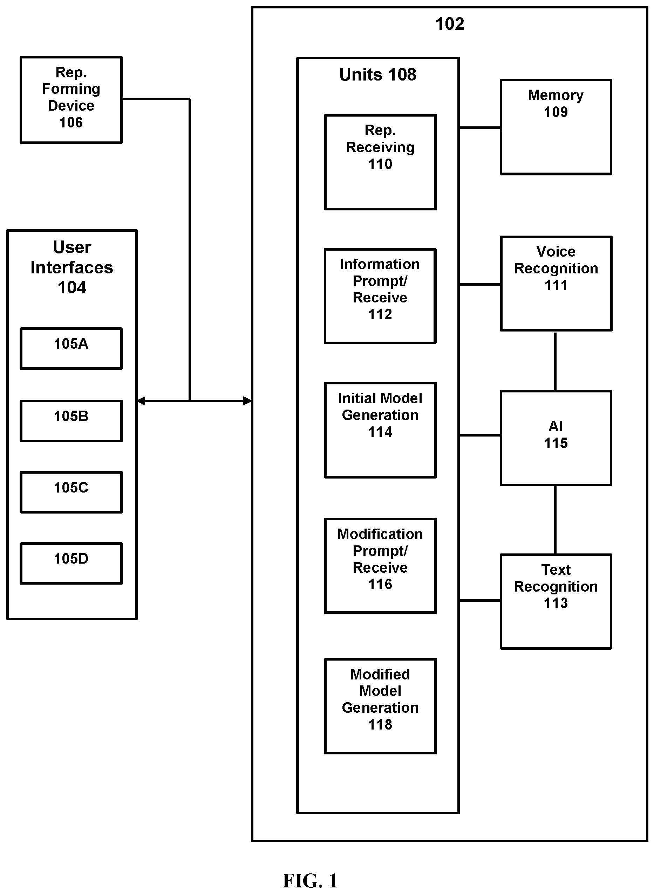

Figure 1 is a conceptual diagram of a system 100 according to embodiments of the invention. The system 100 includes a computer system 102, user interfaces 104 operatively connected to the computer system 102, and a representation forming device 106, as will be described below. The computer system 102 depicted in Figure 1 includes an AI unit 115. But, as described below, the computer system 102 may be operatively connected to another system that provides the AI functionality.

The computer system 102 receives instructions from a user for the creation of a dental prosthesis model, with the computer system 102 including one or more computer devices. Such computers will typically include a central processing unit (CPU) that includes at least one processor. Such computers may also include additional processing structures, such as a graphical processor of a graphical processing unit (GPU). The one or more processors are coupled to one or more memory structures, which may be random access memory (RAM) devices, cache memories, non-volatile or backup memories, read-only memories, etc. In addition, memory may be considered to include memory storage physically located elsewhere in computer, e.g., any cache memory in a processor, as well as any storage capacity used as a virtual memory, e.g., as stored on a mass storage device or on another computer system that is coupled to the computer system 102. It should be noted that a cloud-based architecture could also be used for the computer system 102. As will be appreciated by those skilled in the art, cloud computing is the delivery of services-such as servers, storage, databases, networking, software, analytics, and intelligence-over the internet ("the cloud"). As an example of an embodiment of the invention, a server computer could receive the instructions from the user interfaces and perform the functionalities described herein, or the server computer could be connected to one or more further computers, e.g., through a local network or the Internet, that perform the functionalities described herein together with the server computer.

The computer system 102 will also typically include at least one communication interface comprising input and output components for communicating with external devices. Such a communication interface can be provided by a variety of technologies. For example, the communication interface may include wired communication components, wireless communication components, cellular communication components, Near Field Communication (NFC) components, BLUETOOTH^{®} components, WIFI^{®} components, and other communication components to provide communication via other modalities. In the embodiment depicted in Figure 1, the computer system 102 includes one or more interfaces to communicate with the user interfaces 104.

The computer system 102 may further include one or more data storage devices, with one example memory storage 109 being depicted in Figure 1. The data storage device(s) is configured to record data persistently, where "persistently" or "persistent" refers as to a device's ability to maintain recorded data after loss of power. In some embodiments, the data storage device may correspond to non-disk storage media. For example, the data storage device may be one or more solid-state drives (SSDs), flash memory-based storage, any type of solid-state non-volatile memory, or any other type of non-mechanical storage device. In other implementations, the data storage device may include mechanical or spinning hard disk, such as hard-disk drives (HDD). As noted above, the computer system 102 could also be used in a cloud-based system and thereby access stored data from a remote computer system. That is, the computer system 102 can be operatively linked to a data storage device that is physically separated from the structure of computer system 102. For example, an input/output component of the communication interface of the computer system 102 may be connected to a network through which the computer system 102 receives data from an external data storage device.

It should be appreciated that the computer system 102 typically includes suitable analog and/or digital interfaces between processors and components as are well known in the art. Other hardware environments are contemplated within the context of embodiments of the invention.

The computer system 102 operates (at least in part) under the control of an operating system and executes or otherwise relies upon various computer software applications, components, programs, objects, units, data structures, etc., some of which will be described in greater detail below. Moreover, various applications, components, programs, objects, units, etc. may also execute on one or more processors in another computer coupled to computer system 102 via a network, *e.g.,* in a distributed or client-server computing environment, whereby the processing required to implement the functions of a computer program may be allocated to multiple computers over a network

For simplicity, in the following descriptions with Figure 1, the computer system 102 will be described as a singular device. But, as clear from the foregoing descriptions and as will be clear to one of ordinary skill in the art, the described computer system is but one example of the vast scope of devices and device configurations that could perform the functionalities described herein.

The user interfaces 104 may take a variety of forms, and further user interfaces beyond those depicted in Figure 1 may be used in embodiments of the invention. In the depicted embodiment, the user interfaces include a display device 105A, a keyboard 105B, and mouse 105C. The user interface 105D is a device such as a smartphone, tablet computer, or virtual reality headset (for spatial computing) that is operatively linked to the computer system 102, *e.g.,* though a Wi-Fi or BLUETOOTH^{®} connection. Those skilled in the art will recognize more specific types and variations of user interfaces that may be used. For example, the display device 105A could include a touchscreen and a microphone.

The representation forming device 106 is capable of forming a representation of the dental area in which the dental prosthesis will be placed. One such type of device is a digital impression (DI) scanner, which is often in the form of a handheld intraoral scanner. Such a device captures images of the patient's teeth and oral structures, with the dental professional maneuvering the scanner within the patient's mouth to capture the images from different angles. From the DI scan, a precise three-dimensional digital model of the patient's dentition is formed. The digital model from the DI scan may be input to the dental prosthesis model generation unit(s) of the system (described below). Those skilled in the art will recognize alternative types of dental representation forming devices that could be used in embodiments of the invention. For example, as alternative to a DI scan, a cone beam computed tomography (CBCT) device could be used to generate 3D images of the dental area. In other embodiments, magnetic resonance imaging (MRI) could be used.

As shown in Figure 1, embodiments of the invention include functional units 108 residing in memory device(s) of the computer system 102. Each of the units 108 is computer-executable code that, when executed by the computer's processors, imparts the unit's functionality to the computer system 102. Those skilled in the art will easily recognize the coding languages and techniques that may be used to create the units. It should also be noted that, as discussed above, a distributed computer system could be used in which the units making up the system reside in different computer devices.

The representation receiving unit 110 receives a representation of the dental area in which the dental prosthesis will be used. For example, in embodiments of the invention where the image representation device 106 is a DI scanner, and the representation receiving unit 110 receives the model from the DI scanner. In addition, or in the alternative, the representation receiving unit 110 could receive a representation (model, image, etc.) that was previously taken of the dental area. For example, the representation receiving unit could receive a representation that is stored in the memory 109 of the computer system 102, or the representation receiving unit could receive a representation that is uploaded to the computer system using one of the user interfaces 104.

The computer system 102 includes an initial dental prosthesis information prompting and receiving unit (first receiving unit) 112. This unit 112 is configured to provide prompts to the user to input information about the dental prosthesis to be designed and then receive the information from the user. The prompts provided to the user may take different forms, with one example being text that is displayed on a user interface such as display device 105A. Specific examples of prompt questions given to the user for a crown design are "What tooth is the crown intended for? (Specify tooth number or location.)" "Is the tooth a molar, premolar, incisor, or canine?" "What is the size and shape of the natural tooth?" "What is the reason for placing the crown? (e.g., decay, fracture, cosmetic improvement)" "What is the natural color of the patient's teeth?" and "Is there a preference for tooth color, and do you have a shade guide preference?" Still further, the prompts could present the user with different options if the user's input is unclear.

In this regard, the system functions as an interactive "chatbot" with the dental professional to obtain the information for generating an initial model of the dental prosthesis. The AI unit 115 can be trained in this regard to provide the interactive prompts for the chatbot.

The initial dental prosthesis model information prompting and receiving unit 112 can also receive unprompted instructions from the user regarding the initial model of the dental prothesis. The instructions could relate, for example, to definition of the prosthesis and its parameters (e.g., calculate a crown for 16), descriptions of the final prosthesis shape, indications of an old/young prosthesis design, physical boundary constraints, manufacturing speed, color, orthodontic descriptions, widths, heights, and functional description of the prosthesis (e.g., contacts and angles). Such instructions can be in the form of text and voice commands. Examples of such commands are "create a crown with strong contacts," "create a crown with weak contacts," "create a crown with strong occlusal and weak approximal contacts," "create a front dental prosthesis with an oval shape and minimal wall thickness," and "create a front crown with 15-degree inclination." Note that the ability to use natural language when providing the information to the computer system is convenient and efficient for a dental professional, allowing the professional to interact with the system seamlessly without the need for complex interfaces or manual inputs. It should also be noted that while the method allows for the dental professional to provide detailed specifications as to the dental prosthesis design, such specifications are not required. Rather, a minimalistic instruction alone could suffice such as "create a crown for 20."

To interpret the commands received by initial dental prosthesis model information prompting and receiving unit 112, the computer systems 104 may employ techniques to recognize voice and text commands, using a combination of databases, pattern recognition, and AI. In this regard, the AI unit 115 can be used in conjunction with a voice recognition unit 111 such that algorithms that analyze the acoustic features of spoken words, comparing them to pre-existing patterns stored in databases. These databases consist of vast datasets of phonetic variations and linguistic structures, allowing the computer to identify and interpret spoken commands accurately. Similarly, in using a text recognition unit 113 in conjunction with the AI unit 115, the computer system 103 can use pattern recognition algorithms to decipher written commands by analyzing linguistic patterns, grammar, and contextual cues. The AI can be used to adapt and improve the computer system's recognition capabilities over time. For example, machine learning algorithms, a subset of AI, allow the system to learn from user interactions and continuously refine its understanding of diverse linguistic nuances and context. Note that AI may be included within the voice recognition unit 111 and the text recognition unit 113, or the units may be operatively connected to a separate AI unit 115, as is depicted in Figure 1.

The initial dental prosthesis model information prompting and receiving unit 112 can also be configured to provide follow-up prompts, directed questions, or present options to the user for more information to be used in creating the initial model. For example, when information provided to the unit 112 is unclear, the initial dental prosthesis model information prompting and receiving unit 112 could provide further questions or prompting to the user to clarify aspects of the intended modeled. In this regard, an iterative process can be performed until sufficient information is received for the initial model to be generated.

The initial dental prosthesis model generation unit 114 generates the initial model of the dental prosthesis. As input for creating the model, the generation unit 114 may use the representation of the dental area received from the representation receiving unit 110 and/or the information from the user that is prompted/received by initial dental prosthesis model information prompting and receiving unit 112. As still further input, the initial dental prosthesis model generation unit 114 can used one or more pre-defined parameters for generating the initial model of the dental prosthesis. Such pre-defined parameters could, for example, be stored in the memory 109 or another linked computing device. Examples of parameters in this regard are semantic segmentation of neighboring and antagonistic teeth, semantic segmentation of the gingiva, minimal/maximal allowed thickness of the material the dental prosthesis is to be produced with, contact strength to neighboring teeth, preparation border, tooth number(s), spacer distance from a restoration to the underlying cavity to make room for the composite glue, and the size and direction of an implant. Those skilled in the art will recognize other parameters that may be used.

An artificial intelligence (AI) unit 115 may be used by the initial dental prosthesis generation unit in the generation of the initial model of the dental prosthesis. As discussed above, the AI unit 115 include a large language model and a second model that is designed for producing three-dimensional models of dental prosthesis as an output. As such, the AI system is able to recognize the input and the output an accurate prosthesis model.

It should be noted that the voice recognition unit 111 and text recognition unit 113 may be configured to work in conjunction with the AI unit 115 as well. That is, AI could be used to help interpret voice and/or text commands received from the user. It should also be noted that while the AI unit 115 in shown as a part of the computer system 102 in Figure 1, in other embodiments the computer system 102 could be operatively linked to another system that provides the AI unit.

The initial dental prosthesis model generated by the initial dental prosthesis model generation unit 114 can be in the form of a digital three-dimensional (3D) model. The model may be sent to a user interface such as the display device 105A for viewing by the user. The model contains the information necessary to form the dental prosthesis, e.g., by computer-aided manufacturing. As will be appreciated by those skilled in the art, the model is suitable for producing a dental prosthesis by techniques such a milling and 3D printing.

The computer system 102 further includes a modification prompting and receiving unit (second receiving unit) 116. This unit 116 is configured to provide one or more prompts to the user for information regarding changes that the user wants to make to aspects of the initial dental prosthesis model and to receive the modification information from the user. The modification prompting and receiving unit 116 operates like way that initial dental prosthesis model information prompting and receiving unit 112 operates in that the unit 116 can receive responses to the prompts and other information from the user in the form of voice or text. Such instructions may contain, for example, information on a tooth selection, an area selection, a geometric change, and detailed descriptions of changes to the model. Some specific examples of such commands from the user are "improve contact situation" "on tooth 47 increase the size of the buccal cusps" "deepen the fissures" "smooth the entire occlusion surface" "increase the occlusal contact area" "add a fourth contact area" and "set the mesial approximal contact strength to 100 micrometer." To interpret the commands received from the user, the modification prompting and receiving unit 116 may work in conjunction with the AI unit 115. Once again, the ability to use natural language when providing the information to the computer system is convenient and efficient for a dental professional.

The system 102 may also include a unit (not depicted) allowing for a user to manually make changes to the models of the dental prosthesis that are generated by the system. Such a unit could, for example, provide for CAD design tools as in conventional systems.

A modified dental prosthesis generation unit 118 functions to modify a model of a dental prothesis according to the information received by the modification prompting and receiving unit 116. The model may be a model generated by an initial dental prosthesis generation unit 112. Alternatively, in other methods according to embodiments of the invention, the model could be a model that was previously modified using the modified dental prosthesis generation unit 118, *e.g.,* a model in an iterative process of forming the dental prosthesis design.

To create the modified model, the modified dental prosthesis generation unit 118 may work in conjunction with the AI unit 115 in the manner that the initial dental prosthesis model generation unit 114 works with the AI unit 115.

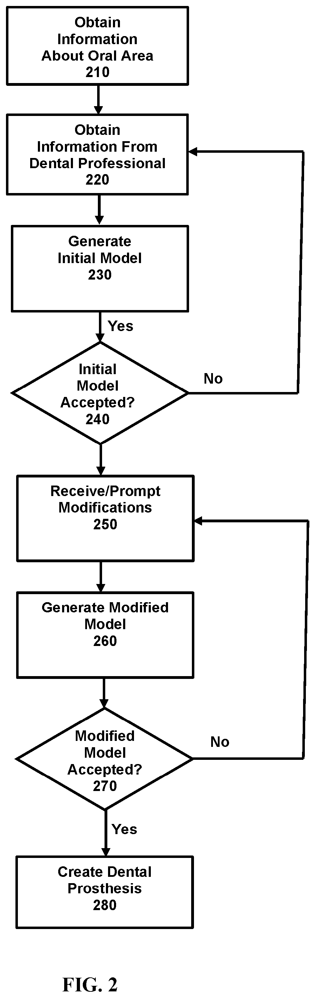

Figure 2 is a flow-diagram of a method of generating a dental prosthesis according to embodiments of the invention. A system as described above may be used to implement the method.

The method begins with a step 210 of obtaining information about the oral area in which the dental prosthesis will be placed. For example, in embodiments of the invention, the information is in the form of a three-dimensional model obtained from a digital impression (DI) scan.

At step 220, information about the dental prosthesis to be generated is obtained from the dental professional. In embodiments of the invention, the information is provided by the dental professional in the form of voice or text that is input to a computer system (as described above). For example, the dental professional could input a voice command indicating the type of prosthesis, the location of the prosthesis, and parameters of the prosthesis. Also in this step, the computer system may provide feedback prompts to user in order to obtain more information or clarification about the dental prosthesis to be modeled. As discussed above, AI may facilitate the interaction with the dental professional.

At step 230, an initial model of the dental prosthesis is generated. The initial model is generated using the information in step 210, the input information about the dental prosthesis received from the dental professional in step 220, and/or pre-defined parameters. In cases where there are no specifications as to the dental prosthesis design, the initial model of dental prosthesis is created using only the pre-defined parameters. In accordance with the discussion above, AI made be used in the generation of the initial model.

Once the initial model for the dental prosthesis is generated, it is presented to the dental professional, e.g., the initial model is displayed on a display device. At step 240, the dental professional evaluates the initial model to decide if the design is acceptably close in the dental professional's opinion to the dental prosthesis that is ultimately desired by the dental professional. If the initial model of the dental prosthesis is not acceptably close in the dental professional's opinion to the dental prosthesis that is desired, the process returns to step 220 to receive further instructions for a new initial dental prosthesis model. If the initial model of the dental prosthesis is acceptably close in the dental professional's opinion to the dental prosthesis that is desired by the dental professional, then the method proceeds to step 250.

At step 250, the dental professional can provide one or more descriptions of modifications to the initial dental prosthesis model. The descriptions may be provided by the dental profession in the form of voice or text. The descriptions of the modification are input into the computer system, which interprets the descriptions (as described above). The system may provide feedback, such as prompts for further clarification from the dental professional or suggestions as to how the model could be modified. The step 250 continues until all of the information for modifying the initial model is received.

At step 260, the system modifies the initial model of the dental prosthesis based on the descriptions of modifications received from the dental professional to thereby generate a modified model of the dental prosthesis. As with generation the initial model of the dental prosthesis, artificial intelligence may be used in generation of the modified model.

At step 270, the modified model of the dental prosthesis is presented to the user for evaluation, e.g., the modified model is displayed on a display device. At this time, the user can accept the modified model, and a dental prosthesis will then be fabricated based on the modified model of the dental prosthesis in step 280. Alternatively, if the user wants additional modifications to be made to the model, then the process returns to step 250 wherein the instructions for the further modifications are provided. Steps 250 and 260 may then be repeated until the dental professional is satisfied with the model of the dental prosthesis.

Those skilled in the art will recognize the technology and techniques available for fabrication of the dental prosthesis from the final design. For example, computer-aided manufacturing (CAM) such as precision milling or 3D printing technologies could be used to form the prosthesis.

In conjunction with conducting the method of generating a dental prosthesis, the AI system may be trained. As discussed above, a large number of training sets can be input to the AI system through daily work of some dental professionals by storing for their speech and/or text input with the AI geometry of the prosthesis model and also the final prosthesis model that dental professional accepts.

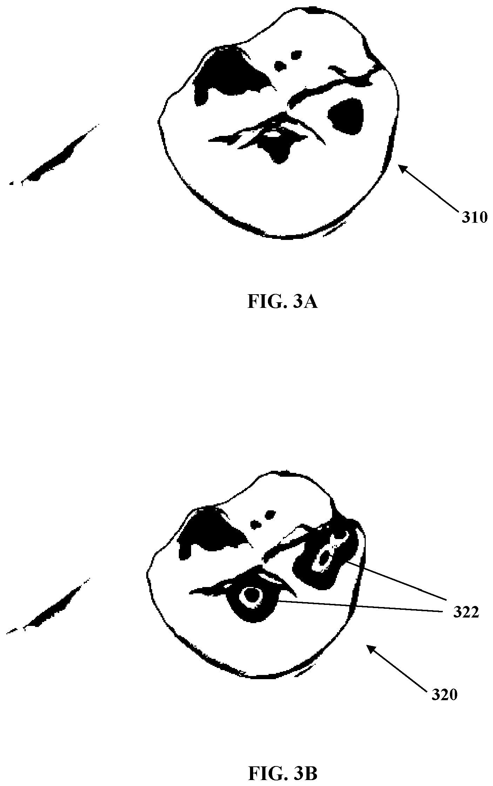

Figures 3A-3G show an example of creating a dental prosthesis model according to embodiments of the invention. In this example, the dental prosthesis is a crown for tooth number 47 (per the universal numbering system for dental notation) in the lower right area of a patient's oral cavity. The figures show the crown model as displayed on the screen of a display device in a system as described above.

The initial crown model 310 is shown in Figure 3A. The initial model 310 is created using the systems, apparatus, and methods described above. That is, the initial model is created using one or more of a representation of the dental area for the crown, information from the user regarding aspects of the crown, and one or more pre-defined design parameters. AI may be used in the generation of the initial crown model 310.

Figure 3B shows a modified crown model 320 formed using the initial crown model 310 and modifications instructions received from the user. In this case, the instruction was a voice or text command to "increase the size of the buccal cusps." Given the instruction, the system modifies the buccal cusps 322 in the design of the crown. To make this modification, the system may consult an AI system, as described above. In comparison to the buccal cusps in the initial crown model 310 in Figure 3A, the area of the buccal cusps 322 is increased in the modified crown model 320.

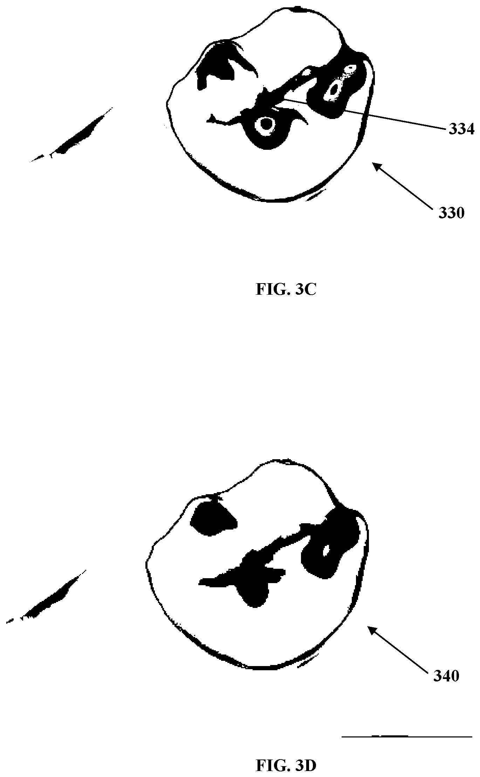

Figure 3C shows a further crown model 330 modified from the crown model 320. The crown model 330 was modified per a voice or text command to "deepen the fissures." To make this modification, the system may again consult an AI system, as described above. In comparison to the fissures in the crown model 320 in Figure 3B, the fissures 334 are deeper in the crown model 330.

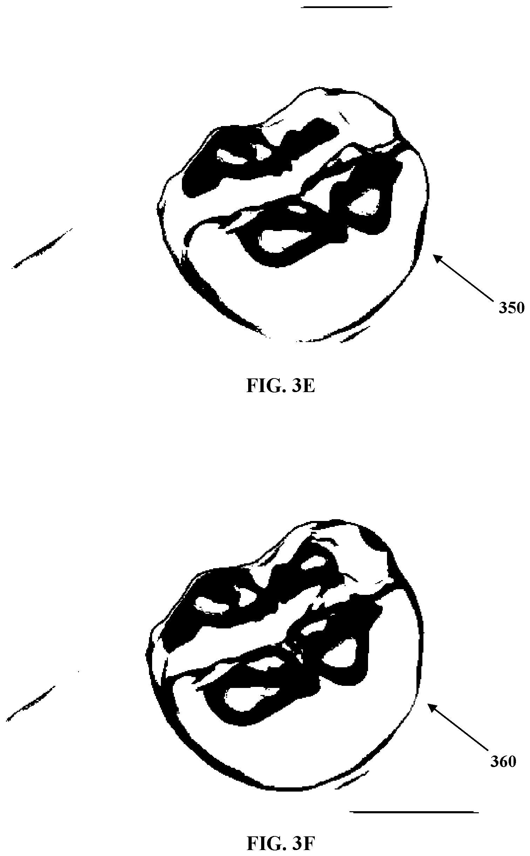

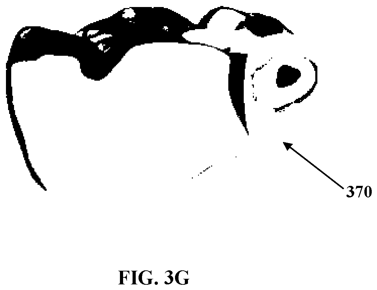

Figures 3D, 3E, 3F, and 3G show still further modifications to the crown model using the same techniques as the modifications depicted in Figures 3B and 3C. To generate the crown model 340 shown in Figure 3D, the voice or text command was to "smooth the entire occlusion surface," and entirety of the occlusion surface is thereby smoother than in the previous crown models. To generate the crown model 350 shown in Figure 3E, the voice or text command was to "increase the occlusal contract area." To generate the crown model 360 shown in Figure 3F, the voice or text command was to "add a fourth contact area." The system thereby automatically determines a best placement for the fourth contact area. AI may again be used to make such a determination. To generate the crown model 370 shown in Figure 3G, the voice or text command was to "set the mesial approximal contact strength to 100 micrometer." Note that in Figure 3F, the view of the crown model 370 has been shifted compared to the views in Figures 3A-3G.

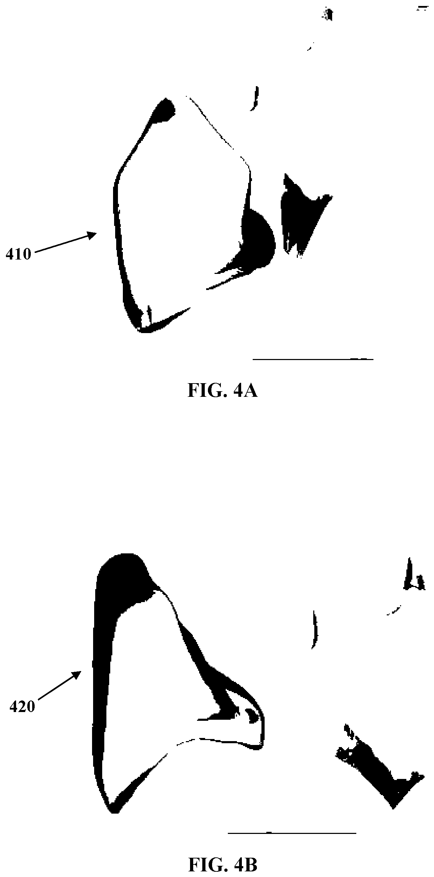

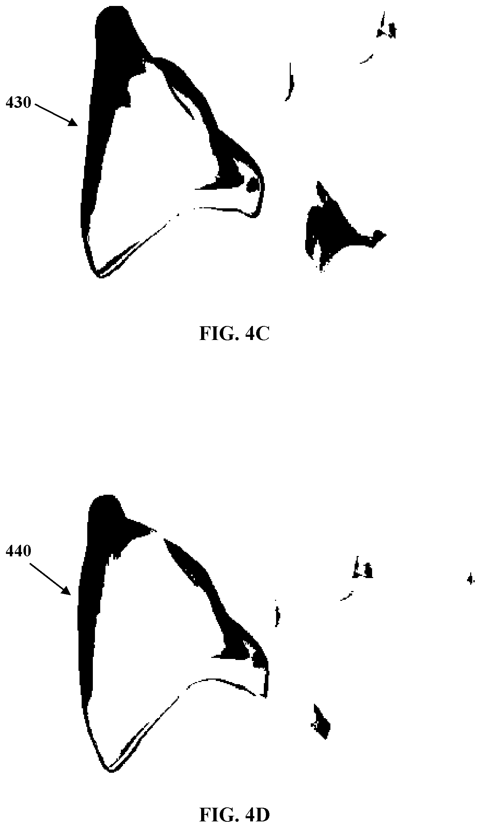

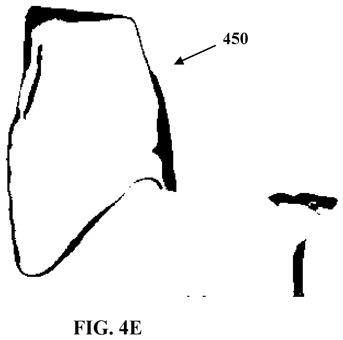

Figures 4A-4E show another example of creating a dental prosthesis model according to embodiments of the invention. In this example, the dental prosthesis is a prosthetic for front tooth number 41 (per the universal numbering system for dental notation). The figures show the prosthetic tooth as displayed on the screen of a display device in a system as described above. Figure 4A shows the initial model of the prosthetic tooth 410. The initial model is created using the systems, apparatus, and methods described above. That is, the initial model is created using one or more of a representation of the dental area for the prosthetic tooth, information from the user regarding aspects of the prosthetic tooth, and one or more pre-defined design parameters.

Figures 4B-4E show modified models of the prosthetic tooth 420-450. The modified model 420 was created from the initial model 410 and a voice or text command to "align incisal angle with neighboring teeth." The modified model 430 was created from the modified model 420 and a voice or text command to "sharpen incisal edges." The modified model 440 was created from the modified model 430 and a voice or text command to "remove concavities on labial surface." The modified model 450 was created from the modified model 440 and a voice or text command to "add more structure to labial surface."

As evident from the foregoing descriptions, systems, apparatuses, and methods according to embodiments of the invention will allow for fast and easy creations of models of dental prostheses. Embodiments of the invention allow dental professionals to specify the design of the models using natural language and text. The ability to receive high-level instructions for creating a model makes the embodiments of the invention much easier to use than conventional systems for generating models. Further, embodiments of the invention in effect provide a "chatbot" that prompts the user for information, such as clarifications as to how a model should be modified. In sum, the embodiments of the invention will save dental professionals valuable time in dental restoration processes.

Further aspects of the apparatuses, systems, and methods described above may be implemented as an article of manufacture such as a non-transitory computer readable storage medium. The non-transitory computer readable storage medium may be readable by a computer and may comprise instructions for causing the computer to perform functions described herein. The non-transitory computer readable storage medium may be implemented by a volatile computer memory, non-volatile computer memory/storage, hard drive, solid-state memory, flash drive, removable disk and/or other media.

The terminology used in the description of the invention herein is for the purpose of describing particular implementations only and is not intended to be limiting of the invention. As used in the description of the invention and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof.

The foregoing description, for purpose of explanation, has been described with reference to specific implementations. However, the illustrative discussions above are not intended to be exhaustive or to limit the invention to the precise forms disclosed. Many modifications and variations are possible in view of the above teachings. The implementations described herein were chosen and described to best explain the principles of embodiments of the invention and its practical applications, to thereby enable others skilled in the art to best utilize embodiments of the invention and various implementations with various modifications as are suited to the particular use contemplated.

## Claims

1. A system for generating a digital model of a dental prosthesis, the system comprising:
at least one processor configured to read out and execute instructions stored in at least one memory to thereby cause the system to function as:
a first receiving unit configured to receive information about the dental prosthesis to be modeled;
an initial dental prosthesis model generation unit configured to generate an initial model of the dental prosthesis using the received information about the dental prosthesis to be modeled;
a second receiving unit configured to receive information for modifying the initial model of the dental prosthesis; and
a modified dental prosthesis model generation unit configured to generate a modified model of the dental prosthesis using the information for modifying the initial model of the dental prosthesis,
wherein the system is configured to receive at least one of the information about the dental prosthesis to be modeled and the information for modifying the initial model of the dental prosthesis in the form of voice or text, and
wherein at least one of the first receiving unit, initial dental prosthesis model generation unit, second receiving unit, and modified dental prosthesis model generation unit is configured to use artificial intelligence when performing functions.

2. A system according to claim 1, wherein the artificial intelligence includes a large language model and a second model configured to produce three-dimensional models of dental prosthesis;
optionally wherein at least one of the initial model of the dental prosthesis and the modified model of the dental prosthesis is produced by the second model of the artificial intelligence.

3. A system according to claim 1 or claim 2, wherein at least one of the first receiving unit and the second receiving unit is configured to output a prompt requesting information to be used to generate the initial model of the dental prosthesis or information to modify the initial model of the dental prosthesis; and/or
wherein at least one of the first receiving unit and the second receiving unit is configured to output a prompt requesting further information to be used to generate the initial model of the dental prosthesis or modify the initial model of the dental prosthesis in response to the received information about the dental prosthesis to be modeled or the received information for modifying the initial model of the dental prosthesis.

4. A system according to any of claims 1-3, wherein the first receiving unit is configured to receive a model generated by a digital impression scanner, and the initial dental prosthesis model generation unit is configured to use the model generated by the digital impression scanner to generate the initial model of the dental prosthesis.

5. A system for generating a digital model of a dental prosthesis, the system comprising:
at least one processor configured to read out and execute instructions stored in at least one memory to thereby cause the system to function as:
a receiving unit configured to receive information in the form of voice or text about the dental prosthesis to be modeled; and
a dental prosthesis model generation unit configured to generate a model of the dental prosthesis based on the information received by the receiving unit, with the model generation unit using artificial intelligence.

6. A system according to claim 5, wherein the artificial intelligence includes a large language model and a second model configured to produce three-dimensional models of dental prosthesis.

7. A system according to claim 5 or claim 6, wherein the first unit is configured to output a prompt requesting information to be used by the dental prosthesis model generation unit to generate the model of the dental prosthesis or to modify the model of the dental prosthesis;
optionally wherein the prompt is a first prompt, and the receiving unit is configured to output a second prompt for further information to be used by the dental prosthesis model generation unit to generate the model of the dental prosthesis or modify the model of the dental prosthesis.

8. A system according to any of claims 5-7, wherein the receiving unit is configured to receive a model generated by a digital impression scanner, and the dental prosthesis model generation unit is configured to use the model generated by the digital impression scanner to generate the model of the dental prosthesis.

9. A method for generating a digital model of a dental prosthesis performed by a computer system, the method comprising:
receiving information about the dental prosthesis to be modeled;
generating an initial model of the dental prosthesis based on the received information about the dental prosthesis to be modeled;
providing a display of the initial model of the dental prosthesis on a display device;
receiving information for modifying the initial model of the dental prosthesis;
generating a modified model of the dental prosthesis based on the information for modifying the initial model of the dental prosthesis; and
providing a display of a modified model of the dental prosthesis on the display device,
wherein at least one of the information about the dental prosthesis and the information for modifying the initial model of the dental prosthesis is received by the computer system in the form of voice or text, and
wherein artificial intelligence is used in at least one of the generation of the initial model of the dental prosthesis and the generation of the modified model of the dental prosthesis.

10. The method according to claim 9, wherein artificial intelligence includes a large language model and a second model configured to produce three-dimensional models of dental prosthesis.

11. The method according to claim 9 or 10, further comprising providing on the display device a prompt requesting the information about the dental prosthesis to be modeled and/or a prompt requesting the information for modifying the initial model of the dental prosthesis;
optionally further comprising providing on the display a request for clarification regarding the information about the dental prosthesis to be modeled and/or a request for clarification regarding the information for modifying the initial model of the dental prosthesis.

12. A method of creating a dental prosthesis comprising:
fabricating the dental prosthesis using the initial model of the dental prosthesis or the modified model of the dental prosthesis generated by the method according to any of claims 9-11.

13. A dental prosthesis made according to the method of claim 12.

14. A non-transitory computer readable storage medium storing computer-readable instruction that cause a processor of a computer system to:
receive information about the dental prosthesis to be modeled;
generate an initial model of the dental prosthesis based on the received information;
provide a display of the initial model of the dental prosthesis on a display device;
receive information for modifying the initial model of the dental prosthesis;
generate a modified model of the dental prosthesis based on the information for modifying the initial model of the dental prosthesis; and
provide a display of a modified model of the dental prosthesis on the display device,
wherein at least one of the information about the dental prosthesis and the information for modifying the initial model of the dental prosthesis is received by the computer system in the form of voice or text, and
wherein artificial intelligence is used in at least one of the generation of the initial model of the dental prosthesis and the generation of the modified model of the dental prosthesis.

15. The non-transitory computer-readable medium according to claim 14, wherein artificial intelligence includes a large language model and a second model configured to produce three-dimensional models of dental prosthesis.
